Europäisches Patentamt

European Patent Office

Office européen des brevets

(11) Publication number: **0 384 535**
**A2**

## (12) EUROPEAN PATENT APPLICATION

(21) Application number: **90200396.1**

(22) Date of filing: **21.02.90**

(51) Int. Cl.⁵: **A61L 2/20**

(30) Priority: **22.02.89 JP 42874/89**

(43) Date of publication of application:
**29.08.90 Bulletin 90/35**

(84) Designated Contracting States:
**DE FR GB NL SE**

(71) Applicant: **Shikoku Kakoki Co., Ltd.**
**10-1, Aza-Nishinokawa Tarohachizu**
**Kitajima-cho**
**Itano-gun Tokushima(JP)**

(72) Inventor: **Akai, Tadao, c/o Shikoku Kakoki Co.**
**Ltd.**
**10-1, Aza-Nishinokawa, Tarohachizu**
**Kitajima-cho, Itano-gun, Tokushima(JP)**
Inventor: **Shibata, Masato, c/o Shikoku Kakoki**
**Co. Ltd.**
**10-1, Aza-Nishinokawa, Tarohachizu**
**Kitajima-cho, Itano-gun, Tokushima(JP)**
Inventor: **Nishino, Hiromichi, c/o Shikoku**
**Kakoki Co. Ltd.**
**10-1, Aza-Nishinokawa, Tarohachizu**
**Kitajima-cho, Itano-gun, Tokushima(JP)**

(74) Representative: **Noz, Franciscus Xaverius, Ir.**
**et al**
**Algemeen Octrooibureau P.O. Box 645**
**NL-5600 AP Eindhoven(NL)**

(54) **Apparatus for supplying hydrogen peroxide for sterilization.**

㉗ An apparatus for supplying hydrogen peroxide to the article to be sterilized, comprising a gasifier for gasifying an aqueous solution of hydrogen peroxide to produce hydrogen peroxide gas, a feeder for supplying the solution to the gasifier in a variable amount, a conveyor for conveying the hydrogen peroxide gas from the gasifier to the article, a sensor for detecting the concentration of the hydrogen peroxide gas being conveyed to produce an electric signal corresponding to the concentration, and a controller for controlling the amount of supply of the solution by the feeder so that the output signal value of the sensor becomes a predetermined set value.

FIG.1

# APPARATUS FOR SUPPLYING HYDROGEN PEROXIDE FOR STERILIZATION

## BACKGROUND OF THE INVENTION

The present invention relates to apparatus for supplying hydrogen peroxide to packaging materials or packaging machines for sterilization.

When hydrogen peroxide is to be supplied to containers which are to be sterilized, an aqueous solution of hydrogen peroxide is usually sprayed from a nozzle, and the atomized solution is entrained by a stream of air and applied to the containers.

It is required that a constant amount of hydrogen peroxide be applied to the containers. For this purpose, there arises a need to recognize the concentration of hydrogen peroxide being conveyed, whereas there is no effective means for directly measuring the concentration of atomized hydrogen peroxide. Accordingly, the concentration of hydrogen peroxide being conveyed is measured indirectly by sampling the hydrogen peroxide during conveyance and measuring the concentration of the samples.

Since atomized hydrogen peroxide partially decomposes into a gas when subjected to heat during conveyance, the state of the atomized hydrogen peroxide being conveyed incessantly changes to result in varying concentrations. The atomized peroxide therefore needs to be sampled frequently in order to detect the gas concentration accurately, but the procedure is cumbersome. Thus, difficulties are encountered in supplying hydrogen peroxide at a predetermined concentration with good stability.

## SUMMARY OF THE INVENTION

The main object of the present invention is to provide an apparatus free of the above problem for supplying hydrogen peroxide for sterilization.

The present invention provides a hydrogen peroxide supplying apparatus which comprises means for gasifying an aqueous solution of hydrogen peroxide to produce hydrogen peroxide gas, supply means for supply ing the aqueous solution of hydrogen peroxide to the gasifying means in a variable amount, means for conveying the hydrogen peroxide gas from the gasifying means to the article to be sterilized, a sensor for detecting the concentration of the hydrogen peroxide gas being conveyed to produce an electric signal corresponding to the concentration, and control means for controlling the amount of supply of the solution by the supply means so that the output signal value of the sensor becomes a predetermined set value.

According to the invention, when the temperature to which hydrogen peroxide is subjected for gasification varies, the mode of decomposition of the peroxide alters to vary the gas concentration and vary the output signal value of the sensor. Based on the output signal, the amount of hydrogen peroxide solution to be supplied by the supply means is so controlled that the sensor output signal value becomes the set value. Consequently, the hydrogen peroxide gas can be supplied at a predetermined concentration with good stability.

## BRIEF DESCRIPTION OF THE DRAWINGS

Fig. 1 is a block diagram showing the construction of an apparatus embodying the invention;

Fig. 2 is a circuit diagram showing control means;

Fig. 3 is a circuit diagram showing another control means;

Fig. 4 is a graph showing the relationship between the amount of hydrogen peroxide aqueous solution supplied dropwise and the gas concentration;

Fig. 5 is a graph showing the relationship between the gas concentration and the amount of hydrogen peroxide applied to the container; and

Fig. 6 is a graph showing the relationship between the gas concentration and the sensor output voltage.

## DESCRIPTION OF THE PREFERRED EMBODIMENTS

Embodiments of the invention will be described below with reference to the drawings.

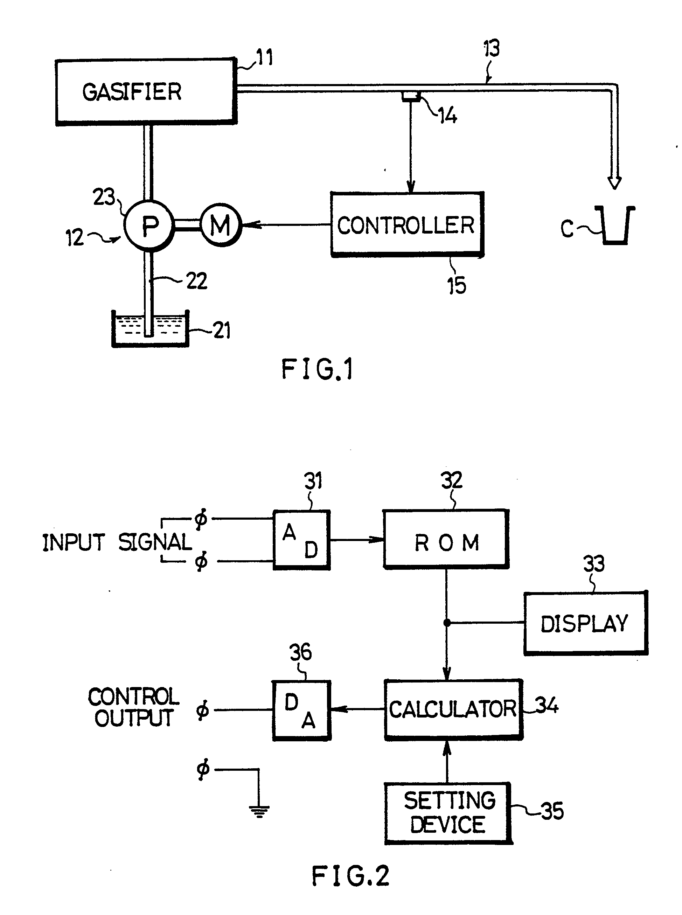

Fig. 1 shows an apparatus for supplying hydrogen peroxide which comprises a unit 11 for producing hydrogen peroxide gas (gasifier), supply means 12 for supplying an aqueous solution of hydrogen peroxide to the gas producing unit 11, means 13 for conveying the hydrogen peroxide gas from the unit 11 to a container C as the article to be sterilized, a sensor 14 for detecting the concentration of the hydrogen peroxide gas being conveyed by contact therewith to produce a signal corresponding to the concentration, and control means 15 for controlling the amount of hydrogen peroxide solution to be supplied by the means 12 so that the output signal value of the sensor 14 becomes a predetermined set value.

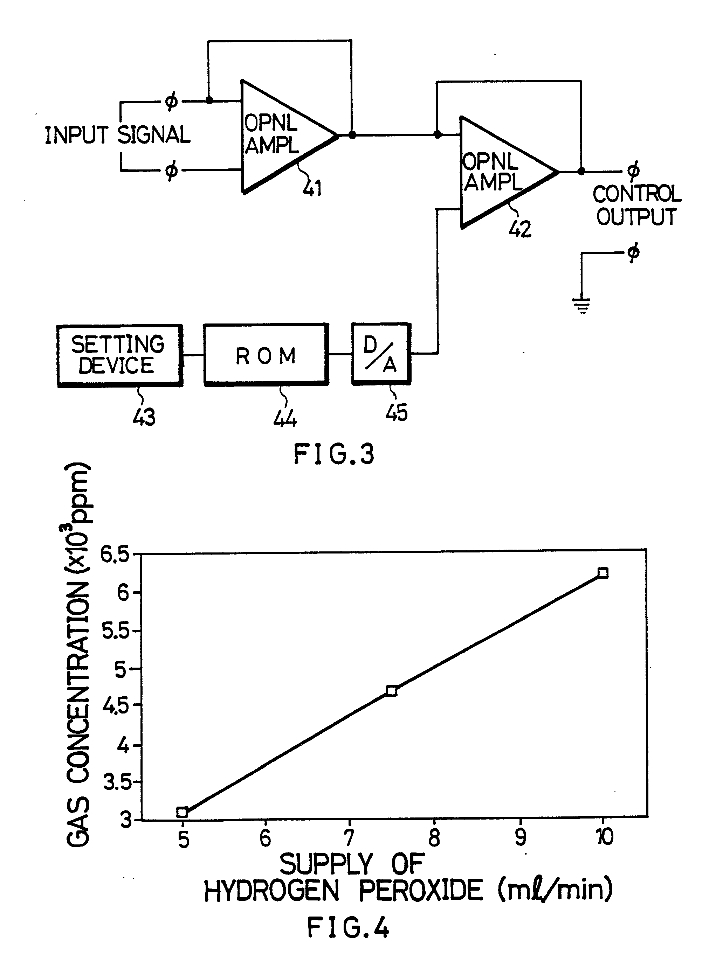

The gas producing unit 11 is adapted to apply the aqueous solution of hydrogen peroxide to a heating member dropwise to vaporize the solution although not shown. Fig. 4 is a graph showing the relationship between the amount of hydrogen peroxide solution applied dropwise and the gas concentration, as established under specified conditions, for example, with respect to the temperature of the heating member and the size of drops. The gas producing unit 11 may be replaced by a unit for spraying the solution into hot air to effect vaporization using two nozzles for the solution and hot air.

The supply means 12 comprises a supply pipe 22 extending from the gas producing unit 11 to a tank 21, and an electric motor 23 provided on the supply pipe 22. The pump 23 is suitably of the peristaltic type capable of forwarding a liquid accurately at an adjustable small rate.

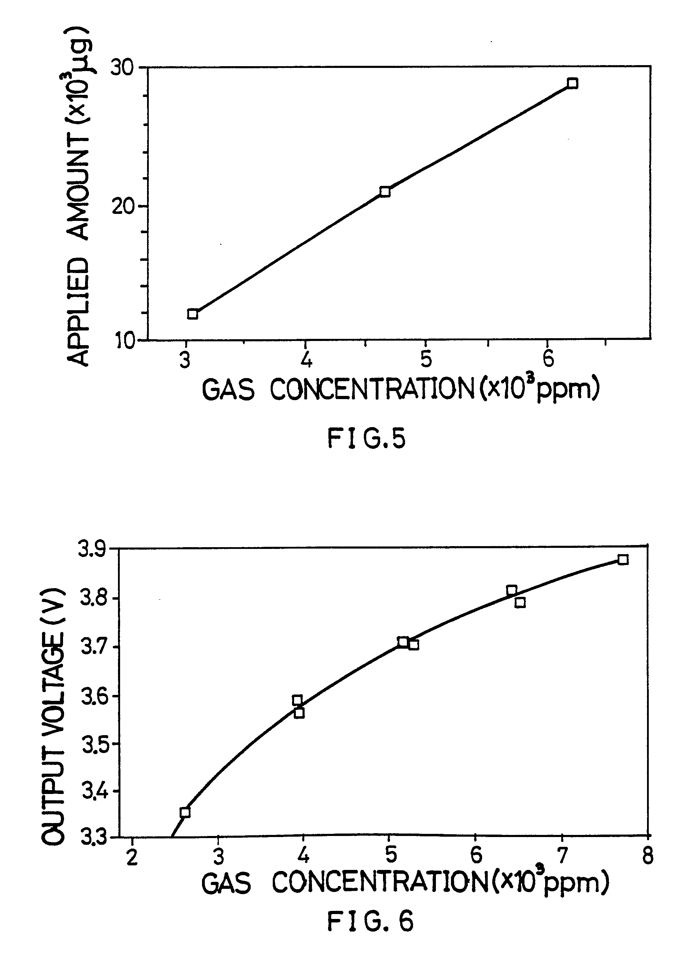

The conveying means 13 comprises conveying air, and a pipe for guiding the air therethrough. The gas conveyed by the means 13 is applied to the container C. Fig. 5 is a graph showing the relationship between the concentration of gas being conveyed and the amount of hydrogen peroxide applied to the container C.

The sensor 14 is a well known gas sensor for use in household gas leakage alarms or for industrial use. Fig. 6 is a graph showing the relationship between the gas concentration and the sensor output voltage, as determined from actual measurements.

Fig. 2 shows a specific example of control means 15.

The sensor 14 produces an analog signal, which is converted by an A/D converter 31 to a digital signal and then fed to a ROM 32. The ROM 32 has stored therein data as to the relationship between the gas concentration and the sensor output voltage shown in Fig. 6. The data determined from the relationship and delivered from the ROM 32 is fed as it is to a display 33 to show the current gas concentration and is also fed to a calculator 34. A predetermined desired gas concentration is set by a setting device 35 and is fed to the calculator 34. The calculator 34 calculates the amount (rate) of hydrogen peroxide solution to be supplied by the supply means 12 based on the output data from the ROM 32 and the set value of the setting means 35 so that the gas concentration measurement becomes the predetermined gas concentration. The output data from the calculator 34 is converted by a D/A converter 36 to analog data, which is used as a control signal for the pump 23.

Fig. 3 shows another specific example of control means.

The output signal from the sensor 14 is amplified by a first operational amplifier 41 and then fed to a second operational amplifier 42. On the other hand, a gas concentration is set by a setting device 43 and fed to a ROM 44, which in turn delivers voltage data corresponding to the gas concentration. The output voltage data is converted by a D/A converter 45 to analog data, which is then fed to the second operational amplifier 42. The second operational amplifier 42 produces a signal corresponding to the deviation of the signal from the sensor 14, from the signal from the setting device 43. The output signal of the amplifier 43 is used as a servo control signal for the pump 23.

## Claims

1. An apparatus for supplying hydrogen peroxide to the article to be sterilized, the apparatus comprising:
   means for gasifying an aqueous solution of hydrogen peroxide to produce hydrogen peroxide gas,
   supply means for supplying the aqueous solution of hydrogen peroxide to the gasifying means in a variable amount,
   means for conveying the hydrogen peroxide gas from the gasifying means to the article to be sterilized,
   a sensor for detecting the concentration of the hydrogen peroxide gas being conveyed to produce an electric signal corresponding to the concentration, and
   control means for controlling the amount of supply of the solution by the supply means so that the output signal value of the sensor becomes a predetermined set value.

2. An apparatus as defined in claim 1 wherein the control means includes a device for setting the concentration of hydrogen peroxide gas to be conveyed, memory means having stored therein comparative data representing a predetermined corresponding relationship between concentrations of hydrogen peroxide gas and output signals of the sensor, read-out means for reading from the memory means data as to the concentration of hydrogen peroxide gas corresponding to the current output signal of the sensor, and means for calculating the amount of hydrogen peroxide aqueous solution to be supplied by the supply means based on the output signal from the setting device and the output signal from the read-out means.

3. An apparatus as defined in claim 1 wherein the supply means includes an electric pump for supplying the aqueous solution of hydrogen peroxide, and the control means includes a device for setting the concentration of hydrogen peroxide gas to be conveyed, memory means having stored therein comparative data representing a predetermined corresponding relationship between concentrations of hydrogen peroxide gas and output signals of the sensor, read-out means for reading from the memory means data as to the output signal of the sensor corresponding to the concentration set by the setting means, a comparator for determining the deviation of the current output signal of the sensor from the output signal of the read-out means, and servo means for controlling the pump so that the output signal of the comparator becomes zero.

4. An apparatus as defined in claim 1 wherein the gasifying means includes a heating member and means for applying the aqueous solution of hydrogen peroxide thereto dropwise.

5. An apparatus as defined in claim 1 wherein the gasifying means includes means for supplying hot air and a nozzle for spraying the aqueous solution of hydrogen peroxide into the hot air.

6. An apparatus as defined in claim 1 wherein the conveying means includes conveying air and a pipe for guiding the air therethrough.

7. An apparatus as defined in claim 2 further comprising a display, and means for showing the output signal of the read-out means on the display.

GASIFIER 11

13

14

23 P M CONTROLLER

12 22

21

15 C

FIG.1

31 32

φ A D ROM

INPUT SIGNAL

φ

33

DISPLAY

36

CONTROL OUTPUT φ D A CALCULATOR 34

φ

SETTING DEVICE 35

FIG.2

INPUT SIGNAL $\phi$

OPNL AMPL 41

OPNL AMPL 42

$\phi$ CONTROL OUTPUT

$\phi$

SETTING DEVICE 43

ROM 44

D/A 45

FIG.3

GAS CONCENTRATION ($\times 10^3$ ppm)

6.5

6

5.5

5

4.5

4

3.5

3

5    6    7    8    9    10

SUPPLY OF
HYDROGEN PEROXIDE (m$\ell$/min)

FIG.4

FIG.5

FIG. 6